# EUROPÄISCHE PATENTSCHRIFT

(11) **EP 0 605 483 B1**
(45) Veröffentlichungstag und Bekanntmachung des Hinweises auf die Patenterteilung: **03.04.1996**
(21) Anmeldenummer: 92919232.6
(22) Anmeldetag: 10.09.1992
(51) Int. Cl.: C09K 3/30, A61K 9/00, A61K 9/12

(54) **TREIBMITTEL FÜR AEROSOLE UND DOSIERAEROSOLE**
PROPELLANT FOR AEROSOLS AND DOSING AEROSOLS
GAZ PROPULSEUR POUR AEROSOLS ET AEROSOLS DOSEURS

(30) Priorität: 27.09.1991 DE 4132176
(43) Veröffentlichungstag der Anmeldung: 13.07.1994
(73) Patentinhaber: IG SPRÜHTECHNIK GMBH, D-78656 Wehr/Baden (DE)
(72) Erfinder: WARNKE, Gieselher, D-7881 Herrischried (DE)
(74) Vertreter: Flaccus, Rolf-Dieter, Dr.
(86) Internationale Anmeldenummer: EP9202086
(87) Internationale Veröffentlichungsnummer: WO9306185

(56) Entgegenhaltungen:
- EP-A- 0 370 632
- EP-A- 0 372 777
- EP-A- 0 504 112
- WO-A-92/08446
- DE-A- 1 945 144
- DE-A- 2 741 882
- DE-A- 3 819 620
- GB-A- 2 240 271

## Beschreibung

Gegenstand der vorliegenden Erfindung ist ein FCKW- und HFKW-freies Treibmittel, dessen Verwendung in Dosieraerosolen, sowie Wirkstoff enthaltende Dosieraerosole mit diesem Treibmittel.

Seit Jahrzehnten werden Aerosol-Druckgaspackungen, kurz Dosieraerosole genannt, unter Verwendung druckverflüssigter Gase oder auch komprimierter Gase als Treibmittel produziert und verwendet. Solche Dosieraerosole bestehen im allgemeinen aus einem Druckbehälter, vorzugsweise aus Metall oder Glas, mit einer Ventilkonstruktion zur Entnahme des Inhalts und dem eigentlichen, zu versprühenden Mittel. Dieses Mittel, auch Wirkstofflösung genannt, kann vielfältiger Natur sein. In den meisten Fällen besteht der Behälterinhalt aus dem zu versprühenden Wirkstoff und einem Treibmittel in Form eines druckverflüssigten Gases. Dieses Gas, eingesetzt werden auch Gasgemische, sollte im Idealfall in jedem Verhältnis mit dem Wirkstoff mischbar sein, so daß eine einzige flüssige Phase entsteht oder es sollte mit Dem Wirkstoff eine gut aufzuschüttelnde Suspension bilden, über der sich eine Gasphase bildet. Je nach enthaltenem Mittel werden diese Dosieraerosole im Kosmetischen und medizinischen Bereich aber auch als Raumspray, Insektizidspray und ähnliches eingesetzt.

Die Treibmittel solcher Aerosole haben besondere Anforderungen zu erfüllen. Sie dürfen auf keinen Fall mit dem Mittel eine Reaktion eingehen. Sie müssen nicht irritierend und nicht toxisch sein. Als besonders geeignet haben sich daher die Fluorchlorkohlenwasserstoffe, kurz FCKW genannt, herausgestellt. Bei diesen Stoffen handelt es sich um gasförmige oder flüssige chemische Stoffe mit besonders günstigen Produkteigenschaften. Sie sind chemisch sehr stabil, unbrennbar und für Mensch und Tier ungiftig. Für antiasthmatische Dosieraerosole werden üblicherweise die Typen R 11, R114 sowie R 12 verwendet. Nachteilig ist ihre ozonabbauende Wirkung, die es aufgrund internationaler Verträge erforderlich macht, die Produktion und die Verwendung dieser Stoffe langfristig ganz einzustellen. Ersatz- und Alternativlösungen sind daher notwendig geworden. Diese Alternativmittel müssen qualitativ den FCKW's vergleichbar sein, insbesondere gesundheitlich unbedenklich und zudem ökologisch verträglich sein. Doch auch die als Ersatzstoffe vielfach propagierten teilhalogenierten FCKW's, sogenannte H-FCKW's und H-FKW's erfüllen nicht diese Anforderungen. Ihre ökologische Verträglichkeit ist zwar besser, doch auch sie weisen immer noch eine Ozonabbaufähigkeit von etwa 5% auf. Hinzu kommt aber noch ein für die Verwendung als Treibmittel für die genannten Zwecke besonderer Nachteil. Zur besseren Dispergierung eines Wirkstoffes im Treibmittel müssen in einigen Fällen, insbesondere bei der Verwendung von antiasthmatischen Wirkstoffen, sogenannte oberflächenaktive Mittel eingesetzt werden. Diese Mittel lösen sich zwar in den FCKW's, nicht aber in den Alternativtreibmitteln des HFKW-Typs auf, so daß hier stets der Zusatz eines polaren Lösungsmittels erforderlich ist.

Aufgabe der vorliegenden Erfindung war daher, ein Treibmittel für Dosieraerosole bereitzustellen, das neben seiner ökologischen Verträglichkeit auch in der Lage sein sollte, oberflächenaktive Stoffe, die üblicherweise beispielsweise antiasthmatischen Dosieraerosolen beigefügt werden, ohne Zusatz eines weiteren Lösungsmittels zu lösen.

Eine weitere Aufgabe bestand darin, Dosieraerosole bereitzustellen, die neben dem erfindungsgemäßen Treibmittel und dem oberflächenaktivem Stoff einen in dieser Mischung enthaltenen Wirkstoff enthalten.

Überraschenderweise wurden diese Aufgaben durch ein Treibmittel , welches aus Isobutan in einer Mischung mit wenigstens einem oberflächenaktiven Mittel besteht, dadurch gekennzeichnet, daß es sich aus 0,05 bis 5,88 Gew.-% eines nicht-ionischen oberflächenaktiven Mittels oder 0,01 - 5,88 Gew.-% Ölsäure und respektive 94,12 bis 99,95 Gew.-% Isobutan oder 94,12 bis 99,99 Gew.-% Isobutan zusammensetzt, gelöst. Dieses Treibmittel ist in der Lage das oberflächenaktive Mittel zu lösen. In Verbindung mit einem speziellen Druckabfüllverfahren ermöglicht dieses Treibmittel die Herstellung umweltneutraler, Wirkstoff enthaltender Dosieraerosole.

Diese guten Eigenschaften bleiben auch erhalten, wenn bis zu ca. 10 Gewichtsprozente (auf Isobutan berechnet) durch Propan ersetzt werden. Die so erzielte Druckerhöhung wirkt sich besonders auf eine verbesserte Vernebelung und Verteilung der suspendierten oder gelösten Wirkstoffe aus.

Die erfindungsgemäß hergestellten Aerosole bilden optisch einwandfreie, leicht aufzuschüttelnde Suspensionen. Dies ist umso überraschender, als man sich bei der Formulierung von FCKW-haltigen Suspensionsaerosolen stets bemühen mußte, die Mischung der Treibmittel so zu wählen, daß sie auf eine Dichte eingestellt werden konnte, die möglichst nahe an der Eigendichte des verwendeten Wirkstoffes lag. Die Dichte von FCKW-Treibgasaerosolen kann daher durchaus zwischen 1,33 und 1,48 liegen, Nur so kann bei FCKW-haltigen Treibgasen eine einwandfreie, leicht aufzuschüttelnde Suspension erzielt werden. Mit dem erfindungsgemäßen Treibmittel in Verbindung mit dem speziellen Druckabfüllverfahren ist ein solches Einstellen überraschenderweise nicht nötig; die Treibgasdichte beträgt stets etwa 0,57, dennoch werden einwandfreie, leicht aufzuschüttelnde Suspensionen erhalten.

Verwendbare oberflächenaktive Mittel sind flüssige, nicht-ionische oberflächenaktive Mittel, wie beispielsweise die Ester oder Teilester der Fettsäuren von 6 bis 22 C-Atomen. Hierzu zählen unter anderem die Capron-, Octan-, Laurin-, Palmitin-, Stearin-, Linol-, Linolen-, Oleostearin- und Ölsäure mit einem aliphatischen mehrwertigen Alkohol oder seinem cyclischen Anhydrid, wie z.B. Ethylenglykol, Glycerin, Erythrit, Arabit, Mannit, Sorbit, die von Sorbit abgeleiteten Anhydride und die Polyoxyethylen- und Polyoxypropylenderivate dieser Ester. Es können gemischte Ester, z.B. gemischte oder natürliche Glyceride wie Olivenöl verwendet werden.

Auch die Ölsäure selbst ist im Sinne der vorliegenden Erfindung als oberflächenaktives Mittel einsetzbar.

Bevorzugte oberflächenaktive Mittel sind neben der Ölsäure die Oleate von Sorbitan, insbesondere Span 85.

Verwendbar ist das erfindungsgemäße Treibmittel wie oben bereits erwähnt sowohl im kosmetischen und medizinischen Bereich als auch in Raumsprays und Insektizidsprays. Besonders geeignet ist es zur Herstellung von Dosieraerosolen, die broncholytische, insbesondere antiasthmatische Wirkstoffe enthalten. Als Wirkstoffe für solche Dosieraerosole kommen die aus FCKW-haltigen, antiasthmatischen Dosieraerosolen bekannten Substanzen in Frage, wobei insbesondere die β-Sympathomimetika wie beispielsweise Isoprenalin, Salbutamol Terbutalin, Fenoterol und Clenbuterol zu nennen sind. Von dem Dinatriumsalz der Cromoglicinsäure, auch DNCG genannt, einer ebenfalls antiasthmatischen, broncholytisch wirkenden Substanz, das insbesondere zur Prophylaxe eines Asthmaanfalls eingesetzt wird, ist bekannt, daß es wegen der großen Polarität nur schlecht resorbiert wird. Bei seiner Verwendung in Aerosolen muß es daher in mikronisierter Form und vorzugsweise mit einem oberflächenaktiven Stoff formuliert werden. Gerade für diesen Wirkstoff hat sich die erfindungsgemäße Treibmittelmischung als besonders vorteilhaft herausgestellt. Auch das Broncholytikum Reproterol kann eingesetzt werden. Neben den Monosubstanzen können auch ihre Mischungen eintgesetzt werden, wobei insbesondere DNCG mit Reproterol bevorzugt ist.

Geeignete Treibmittelmischungen setzen sich zusammen aus 0,05 bis 5,88 Gew.% des nicht ionischen oberflächenaktiven Mittels und 94,12 bis 99,95 Gew.% Isobutan. Bei Verwendung des besonders geeigneten, nicht ionischen oberflächenaktiven Mittels Span 85 zur Herstellung der erfindungsgemäßen Dosieraerosole haben sich folgende Rezepturbilder als vorteilhaft herausgestellt:

### DNCG-DA:

| | |
|---|---|
| DNCG | 1,0 % - 10,0 % |
| Span 85 | 0,5 % - 5,0 % |
| Isobutan | 98,5 % - 85,0 % |

### DNCG + Reproterol-DA:

| | |
|---|---|
| DNCG | 1,0 % - 10,0 % |
| Reproterol | 0,5 % - 5,0 % |
| Span 85 | 0,5 % - 5,0 % |
| Isobutan | 98,0 % - 80,0 % |

### Fenoterol-DA:

| | |
|---|---|
| Fenoterol | 0,20 % - 1,0 % |
| Span 85 | 0,05 % - 0,5 % |
| Isobutan | 99,75 % - 98,5 % |

### Terbutalin-DA:

| | |
|---|---|
| Terbutalin | 0,5 % - 3,0 % |
| Span 85 | 0,5 % - 5,0 % |
| Isobutan | 99,0 % - 92,0 % |

Besonders bevorzugt sind die folgenden Aerosolmischungen:

1 Aerosoldose enthält:

### DNCG-DA:

| | |
|---|---|
| DNCG | 3,35 % ( 200 mg) |
| Span 85 | 2,35 % ( 140 mg) |
| Isobutan | 94,30 % (5630 mg) |

### DNCG + Reproterol-DA:

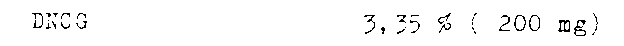

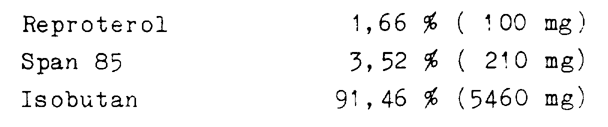

### Fenoterol-DA:

| | |
|---|---|
| Fenoterol | 0,71 % ( 60 mg) |
| Span 85 | 0,18 % ( 15 mg) |
| Isobutan | 99,11 % (8395 mg) |

### Terbutalin-DA:

| | |
|---|---|
| Terbutalin | 1,66 % ( 100 mg) |
| Span 85 | 2,32 % ( 140 mg) |
| Isobutan | 96,03 % (5800 mg) |

Bei der Verwendung von Ölsäure als oberflächenaktives Mittel werden bevorzugt 0,01 bis 0,11 Gew.% eingesetzt. Als vorteilhaft hat sich bei Salbutamol als Wirkstoff das folgende Rezepturbild herausgestellt:

### Salbutamol-DA:

| | |
|---|---|
| Salbutamol | 0,10 % - 0,5 % |
| Ölsäure | 0,01 % - 0,1 % |
| Isobutan | 99,89 % - 99,4 % |

Besonders bevorzugt ist die Aerosolmischung:

1 Aerosoldose enthält:

### Salbutamol-DA:

| | |
|---|---|
| Salbutamol | 0,35 % ( 30 mg) |
| Ölsäure | 0,03 % ( 3 mg) |
| Isobutan | 99,62 % (8550 mg) |

Die erfindungsgemäßen Aerosole können durch Mischen der verschiedenen Bestandteile unter Bedingungen, bei denen das Treibmittel und das oberflächenaktive Mittel flüssig und der Wirkstoff in fester Phase vorliegt, hergestellt werden.

Die Wirkstoffsuspension wird durch das Ventil unter Druck in die verclinchte Dose, die zu Beginn des Füllvorganges Raumtemperatur hat, gefüllt. Die Suspension hat eine Temperatur von ca. -8 bis -10 °C. Anschließend wird mit dem Treibmittel nachgefüllt und so das Ventil gleichzeitig gereinigt.

Die erfindungsgemäßen Dosieraerosole können bei der Behandlung von Mensch und Tier, insbesondere bei der Behandlung allergischer Erkrankungen der Luftwege wie Asthma oder allergischer Rhinitis (Heuschnupfen), vorzugsweise durch orale oder nasale Inhalation, eingesetzt werden.

## Patentansprüche

1. Dosieraerosol mit einem Treibmittel, welches aus Isobutan in einer Mischung mit wenigstens einem oberflächenaktiven Mittel besteht, dadurch gekennzeichnet, daß es sich aus 0,05 bis 5,88 Gew.-% eines nicht-ionischen oberflächenaktiven Mittels oder 0,01 - 5,88 Gew.-% Ölsäure und respektive 94,12 bis 99,95 Gew.-% Isobutan oder 94,12 bis 99,99 Gew.-% Isobutan zusammensetzt und als oberflächenaktives Mittel vorzugsweise Oleate des Sorbitan, besonders bevorzugsweise Span 85, enthält, wobei bis zu 10 Gew.-% des Isobutans durch Propan ersetzt sein können.

2. Dosieraerosol nach Anspruch 1, dadurch gekennzeichnet, daß es als oberflächenaktives Mittel Ölsäure enthält.

3. Dosieraerosol nach einem der Ansprüche 1 oder 2, dadurch gekennzeichnet, daß bis zu 10 Gew.-% des Isobutans durch Propan ersetzt sind.

4. Dosieraerosol nach einem der Ansprüche 1-3, dadurch gekennzeichnet, daß es aus Treibmittel und einem broncholytischen Wirkstoff besteht.

5. Dosieraerosol nach Anspruch 4, dadurch gekennzeichnet, daß es aus Treibmittel und einem broncholytischen Wirkstoff aus der Reihe der β-Sympathomimetika besteht.

6. Dosieraerosol nach Anspruch 4, dadurch gekennzeichnet, daß es aus Treibmittel und aus Salbutamol, Fenoterol, Terbutalin, Reproterol, DNCG oder dessen Mischungen besteht.

7. Dosieraerosol gemäß Anspruch 4, dadurch gekennzeichnet, daß es dem Rezepturbild:
| | |
|---|---|
| Fenoterol | 0,20 % - 1,0 % |
| Span 85 | 0,05 % - 0,5 % |
| Isobutan | 99,75 % - 98,5 % |
entspricht.

8. Dosieraerosol nach Anspruch 4, dadurch gekennzeichnet, daß es dem Rezepturbild:
| | |
|---|---|
| Terbutalin | 0,5 % - 3,0 % |
| Span 85 | 0,5 % - 5,0 % |
| Isobutan | 99,0 % - 92,0 % |
entspricht.

9. Dosieraerosol nach Anspruch 4, dadurch gekennzeichnet, daß es dem Rezepturbild:
| | |
|---|---|
| DNCG | 1,0 % - 10,0 % |
| Span 85 | 0,5 % - 5,0 % |
| Isobutan | 98,5 % - 85,0 % |
entspricht.

10. Dosieraerosol nach Anspruch 4, dadurch gekennzeichnet, daß es dem Rezepturbild:
| | |
|---|---|
| DNCG | 1,0 % - 10,0 % |
| Reproterol | 0,5 % - 5,0 % |
| Span 85 | 0,5 % - 5,0 % |
| Isobutan | 98,0 % - 80,0 % |
entspricht.

11. Dosieraerosol nach Anspruch 4, dadurch gekennzeichnet, daß es dem Rezepturbild:
| | |
|---|---|
| Salbutamol | 0,10 % - 0,5 % |
| Ölsäure | 0,01 % - 0,1 % |
| Isobutan | 99,89 % - 99,4 % |
entspricht .

12. Dosieraerosol nach Anspruch 4, dadurch gekennzeichnet, daß es aus
| | |
|---|---|
| Fenoterol | 0,71 % |
| Span 85 | 0,18 % |
| Isobutan | 99,11 % |
besteht.

13. Dosieraerosol nach Anspruch 4, dadurch gekennzeichnet, daß es aus
| | |
|---|---|
| Terbutalin | 1,66 % |
| Span 85 | 2,32 % |
| Isobutan | 96,03 % |
besteht.

14. Dosieraerosol nach Anspruch 4, dadurch gekennzeichnet, daß es aus
| | |
|---|---|
| DNCG | 3,35 % |
| Span 85 | 2,35 % |
| Isobutan | 94,30 % |
besteht.

15. Dosieraerosol nach Anspruch 4, dadurch gekennzeichnet, daß es aus
| | |
|---|---|
| DNCG | 3,35 % |
| Reproterol | 1,66 % |
| Span 85 | 3,52 % |
| Isobutan | 91,46 % |
besteht.

16. Dosieraerosol nach Anspruch 4, dadurch gekennzeichnet, daß es aus
| | |
|---|---|
| Salbutamol | 0,35 % |
| Ölsäure | 0,03 % |
| Isobutan | 99,62 % |
besteht.

17. Dosieraerosol nach einem der Ansprüche 4 bis 16, dadurch gekennzeichnet, daß bis zu 10 % des Isobutans durch Propan ersetzt sind.

18. Dosieraerosol nach einem der Ansprüche 4 bis 17, zur Behandlung allergischer Erkrankungen bei Mensch und Tier, vorzugsweise zur Inhalationsbehandlung allergischer Erkrankungen der Luftwege.

19. Dosieraerosol nach einem der Ansprüche 4 bis 17, zur Behandlung von Asthma oder allergischer Rhinitis.

## Claims

1. A controlled dosage aerosol having a propellant consisting of isobutane in a mixture with at least one surface-active agent, characterized in that it is composed of 0.05 to 5.88%-wt. of a non-ionic surface-active agent or 0.01 to 5.88%-wt. oleic acid and, respectively, 94.12 to 99.95%-wt. isobutane or 94.12 to 99.99%-wt isobutane, and comprises as surface-active agent preferably oleates of sorbitan, particularly preferred Span 85, up to 10% of the isobutane being able to be replaced by propane.

2. The controlled dosage aerosol according to claim 1 ch aracterized in that it comprises oleic acid as surfactant.

3. The controlled dosage aerosol according to any one of claims 1 to 2 characterized in that up to 10%-wt. of isobutane are replaced by propane.

4. The controlled dosage aerosol according to any one of claims 1 to 3 characterized in that it consists of a propellant and a broncholytic active substance.

5. The controlled dosage aerosol according to claim 4 characterized in that it consists of a propellant and of a broncholytic active substance of the family of β-sympathomimetics.

6. The controlled dosage aerosol according to claim 4 characterized in that it consists of a propellant and of salbutamol, fenoterol, terbutaline, reproterol, DSCG or its mixtures.

7. The controlled dosage aerosol according to claim 4 characterized in that it corresponds to the formulation:

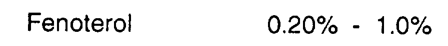

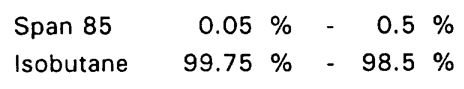

8. The controlled dosage aerosol according to claim 4 characterized in that it corresponds to the formulation:
| | |
|---|---|
| Terbutaline | 0.5 % - 3.0 % |
| Span 85 | 0.5 % - 5.0 % |
| Isobutane | 99.0 % - 92.0 % |

9. The controlled dosage aerosol according to claim 4 characterized in that it corresponds to the formulation:
| | |
|---|---|
| DSCG | 1.0 % - 10.0 % |
| Span 85 | 0.5 % - 5.0 % |
| Isobutane | 98.5 % - 85.0 % |

10. The controlled dosage aerosol according to claim 4 characterized in that it corresponds to the formulation:
| | |
|---|---|
| DSCG | 1.0 % - 10.0 % |
| Reproterol | 0.5 % - 5.0 % |
| Span 85 | 0.5 % - 5.0 % |
| Isobutane | 98.0 % - 80.0 % |

11. The controlled dosage aerosol according to claim 4 characterized in that it corresponds to the formulation:
| | |
|---|---|
| Salbutamol | 0.10 % - 0.5 % |
| Oleic acid | 0.01 % - 0.1 % |
| Isobutane | 99.89 % - 99.4 % |

12. The controlled dosage aerosol according to claim 4 characterized in that it consists of:
| | |
|---|---|
| Fenoterol | 0.71 % |
| Span 85 | 0.18 % |
| Isobutane | 99.11 % |

13. The controlled dosage aerosol according to claim 4 characterized in that it consists of:
| | |
|---|---|
| Terbutaline | 1.66 % |
| Span 85 | 2.32 % |
| Isobutane | 96.03 % |

14. The controlled dosage aerosol according to claim 4 characterized in that it consists of:
| | |
|---|---|
| DSCG | 3.35 % |
| Span 85 | 2.35 % |
| Isobutane | 94.30 % |

15. The controlled dosage aerosol according to claim 4 characterized in that it consists of:
| | |
|---|---|
| DSCG | 3.35 % |
| Reproterol | 1.66 % |
| Span 85 | 3.52 % |
| Isobutane | 91.46 % |

16. The controlled dosage aerosol according to claim 4 characterized in that it consists of:
| | |
|---|---|
| Salbutamol | 0.35 % |
| Oleic acid | 0.03 % |
| Isobutane | 99.62 % |

17. The controlled dosage aerosol according to any one of claims 4 to 16 characterized in that up to 10% of isobutane are replaced by propane.

18. The controlled dosage aerosol according to any one of claims 4 to 17 for the treatment of allergic diseases in man and animal, preferably in the inhalation treatment of allergic diseases of the respiratory tract.

19. The controlled dosage aerosol according to any one of claims 4 to 17 for the treatment of asthma or allergic rhinitis.

## Revendications

1. Aérosol doseur avec un propulseur, constitué d'isobutane en mélange à au moins un agent surfactif, caractérisé en ce qu'il se compose de 0,05 à 5,88% en poids d'un agent surfactif non ionique, ou de 0,01 à 5,88% en poids d'acide oléique et de respectivement 94,12 à 99,95% en poids d'isobutane, ou de 94,12 à 99,99% en poids d'isobutane et contient, à titre d'agent surfactif, de préférence, des oléates du sorbitan, de manière tout particulièrement avantageuse le span 85, où jusqu'à 10% en poids de l'isobutane peuvent être remplacés par du propane.

2. Aérosol doseur suivant la revendication 1, caractérisé en ce qu'il contient de l'acide oléique à titre d'agent surfactif.

3. Aérosol doseur suivant l'une quelconque des revendications 1 et 2, caractérisé en ce que jusqu'à 10% de l'isobutane sont remplacés par du propane.

4. Aérosol doseur suivant l'une quelconque des revendications 1 à 3, caractérisé en ce qu'il se compose d'un propulseur et d'un principe actif broncholitique.

5. Aérosol doseur suivant la revendication 4, caractérisé en ce qu'il se compose d'un propulseur et d'un principe actif broncholitique appartenant à la série des β-sympathicomimétiques.

6. Aérosol doseur suivant la revendication 4, caractérisé en ce qu'il se compose d'un propulseur et de salbutamol, de fénotérol, de terbutaline, de réprotérol, de DNCG, ou de leurs mélanges.

7. Aérosol doseur suivant la revendication 4, caractérisé en ce qu'il correspond à la formule suivante :

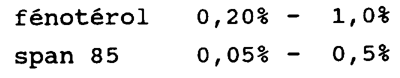

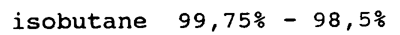

8. Aérosol doseur suivant la revendication 4, caractérisé en ce qu'il correspond à la formule suivante :
| | |
|---|---|
| terbutaline | 0,5 % - 3,0 % |
| span 85 | 0,5 % - 5,0 % |
| isobutane | 99,0 % - 92,0 % |

9. Aérosol doseur suivant la revendication 4, caractérisé en ce qu'il correspond à la formule suivante :
| | |
|---|---|
| DNCG | 1,0 % - 10,0 % |
| span 85 | 0,5 % - 5,0 % |
| isobutane | 98,5 % - 85,0 % |

10. Aérosol doseur suivant la revendication 4, caractérisé en ce qu'il correspond à la formule suivante :
| | |
|---|---|
| DNCG | 1,0 % - 10,0 % |
| réprotérol | 0,5 % - 5,0 % |
| span 85 | 0,5 % - 5,0 % |
| isobutane | 98,0 % - 80,0 % |

11. Aérosol doseur suivant la revendication 4, caractérisé en ce qu'il correspond à la formule suivante :
| | |
|---|---|
| salbutamol | 0,10 % - 0,5 % |
| acide oléique | 0,01 % - 0,1 % |
| isobutane | 99,89 % - 99,4 % |

12. Aérosol doseur suivant la revendication 4, caractérisé en ce qu'il correspond à la formule suivante :
| | |
|---|---|
| fénotérol | 0,71 % |
| span 85 | 0,18 % |
| isobutane | 99,11 % |

13. Aérosol doseur suivant la revendication 4, caractérisé en ce qu'il correspond à la formule suivante :
| | |
|---|---|
| terbutaline | 1,66 % |
| span 85 | 2,32 % |
| isobutane | 96,03 % |

14. Aérosol doseur suivant la revendication 4, caractérisé en ce qu'il correspond à la formule suivante :
| | |
|---|---|
| DNCG | 3,35 % |
| span 85 | 2,35 % |
| isobutane | 94,30 % |

15. Aérosol doseur suivant la revendication 4, caractérisé en ce qu'il correspond à la formule suivante :
| | |
|---|---|
| DNCG | 3,35 % |
| réprotérol | 1,66 % |
| span 85 | 3,52 % |
| isobutane | 91,46 % |

16. Aérosol doseur suivant la revendication 4, caractérisé en ce qu'il correspond à la formule suivante :
| | |
|---|---|
| salbutamol | 0,35 % |
| acide oléique | 0,03 % |
| isobutane | 99,62 % |

17. Aérosol doseur suivant l'une quelconque des revendications 4 à 16, caractérisé en ce que jusqu'à 10% de l'isobutane sont remplacés par du propane.

18. Aérosol doseur suivant l'une quelconque des revendications 4 à 17, pour le traitement de maladies allergiques chez l'homme et chez l'animal, de préférence, pour le traitement par inhalation de maladies allergiques des voies respiratoires.

19. Aérosol doseur suivant l'une quelconque des revendications 4 à 17, pour le traitement de l'asthme ou de la rhinite allergique.
